(19) Europäisches Patentamt / European Patent Office / Office européen des brevets

(11) **EP 3 159 689 A1**

(12) **EUROPEAN PATENT APPLICATION**

(43) Date of publication:
26.04.2017 Bulletin 2017/17

(51) Int Cl.:
*G01N 33/28* (2006.01)

(21) Application number: 15290274.8

(22) Date of filing: 21.10.2015

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(71) Applicant: **Geoservices Equipements
95971 Roissy en France (FR)**

(72) Inventors:
• **Colombel, Emilie
95971 Roissy (FR)**
• **Breviere, Jerome
95971 Roissy (FR)**

(74) Representative: **Leonori, Céline
Etudes et Productions Schlumberger
Intellectual Property Department
1, rue Henri Becquerel
B.P. 202
92142 Clamart Cedex (FR)**

(54) **METHOD FOR DETERMINING THE QUANTITY OF AT LEAST ONE GAS COMPOUND IN A DRILLING FLUID WITHOUT ON-SITE CALIBRATION**

(57) The disclosure relates to a method for determining the content of at least one gas compound contained in a drilling fluid having a set of physical properties, the method comprising:
- extracting out of a sample of the drilling fluid a gas fraction of the or each gas compound;
- measuring a representative information ($y(i)$) of the or each gas fraction of each gas compound;
- obtaining, for the or each gas compound a correcting factor ($<p(i)>$) relating the measured information ($y(i)$) for the gas fraction of each compound to the content of said compound in the drilling fluid;
wherein the estimated correcting factor ($<p(i)>$) used for the or each gas compound is obtained on the basis of a set of previous correcting factors ($p_0(i)$), previously obtained from calibration of reference drilling fluids, each of the reference drilling fluids having at least one physical property substantially identical to the corresponding physical property of the extracted drilling fluid.

FIG.1

EP 3 159 689 A1

**Description**

The present invention concerns a method for determining the content of several gas compounds contained in a drilling fluid. During the drilling of a petroleum or gas well, it is known how to perform an analysis of the gas compounds contained in the drilling fluid emerging from the well, this fluid being commonly designated as "drilling mud". This analysis gives the possibility of reconstructing the geological succession of the crossed formations during the drilling and is involved in the determination of the possibilities of exploiting encountered fluid deposits.

This analysis performed continuously comprises two main phases. A first phase consists of continuously sampling the drilling fluid in circulation in the conduit, and then of bringing it into an extraction enclosure where a certain number of compounds carried by the drilling fluid (for example hydrocarbon compounds, carbon dioxide, hydrogen sulfide, helium and nitrogen) are extracted from the mud as a gas.

A second phase consists of transporting the extracted gases towards an analyzer where these gases are described and in certain cases quantified. For extracting the gases from the mud, a degasser with mechanical stirring as described in FR 2 799 790 is frequently used. The gases extracted from the mud, mixed with a carrier gas introduced into the degasser are conveyed by suction through a gas extraction conduit up to an analyzer which allows quantification of the extracted gases. With such a device it is possible to significantly and specifically extract the very volatile gases present in the mud, for example $C_1$-$C_5$ hydrocarbons, notably when it is used with a device for heating the drilling mud, placed upstream from the degasser or in the latter.

The extraction, In the degasser, does not enable to extract the total amount of compounds present in the mud and the extraction efficiency, defined as the amount of an extracted compound referred to the total amount of this same compound initially contained in the mud, depends on the nature of the compound. It is therefore known how to empirically correct the measurement carried on the gas fraction extracted for each compound with a correction factor depending on the compound, in order to provide an estimate of the actual content of the compound in the drilling mud.

In order to improve the accuracy of the measurement, EP-A-1 710 575 describes a method for obtaining a correcting factor enabling to calibrate the extractor wherein a same calibration sample of the drilling fluid, containing the different compounds to be extracted, successively undergoes several extraction stages in the degasser, the amount of extracted gas being measured at each extraction stage. On the basis of the gas fractions measured at each extraction stage for each compound, a correction factor relating the content of a given compound to the measured fraction during a first extraction stage in the degasser may be determined experimentally for each compound. With such a method the accuracy of the measurement may be considerably improved.

This calibration operation may be time-consuming as it requires finding a suitable sample containing a sufficient amount of compounds to yield accurate results, performing multiple extractions for each sample, and eventually having to perform several calibrations due to the drilling fluid's nature changing radically during the drilling process, at the wellsite.

The disclosure relates to a method for determining, in a simple way, the content of a plurality of compounds contained in a drilling fluid.

More specifically, the disclosure relates to a method comprising:

- extracting out of a sample of the drilling fluid a gas fraction of each gas compound;
- measuring a representative information of each gas fraction of each gas compound; .
- obtaining, for each gas compound a correcting factor relating the measured information for the gas fraction of each compound to the content of said compound in the drilling fluid.

The method also comprises obtaining the estimated correcting factor used for the or each gas compound on the basis of a set of previous correcting factors, previously obtained from calibration of reference drilling fluids, each of the reference drilling fluids having a physical property substantially identical to the corresponding physical property of the extracted drilling fluid.

The method according to the disclosure may comprise one or more of the following features, taken individually or according to any technically possible combination(s):

- the method comprises determining the set of previous correcting factors;
- the set of previous correcting factors is chosen so as to have been calibrated for the same gas compound;
- the physical property relates to the composition of the drilling fluid, the reference drilling fluids being of the same type as the extracted drilling fluid, chosen among a water-based mud, an oil-based mud or a synthetic-based mud;
- the physical property is density or a rheological property such as viscosity, and the difference between the value of the physical property for each of the reference drilling fluids and the value of the corresponding physical property for the extracted drilling fluid is situated in a predetermined range;
- the method comprises measuring at least a physical property of the drilling fluid;
- the estimated correcting factor for a gas compound corresponds to the average value calculated over the set of previous correcting factors related to the same compound;
- the method comprises providing an initial set of previous correcting factors obtained from a database, determining a number of abnormal values from this initial set and determining the set of previous correcting factors used for calculating the estimated correcting factors by excluding the abnormal values;
- the correcting factor tied to a single representative compound out of a set of previous correcting factors in a previous abnormal operation is taken into account to determine if the value is abnormal, and In that case, correcting factors values from the same set of correcting factors arising from the same previous operation are excluded;
- the extraction is implemented on an enclosure comprising a stirrer for stirring the drilling fluid, with chosen extraction conditions comprising:
- a flow rate of injection of the drilling fluid in the enclosure $Q_m$,
- an average volume of drilling fluid in the enclosure $V_m$,
- a volume of overhead gas in the enclosure $V_g$,
- a flow rate of gas extracted from the drilling fluid $Q_p$,

the set of previous correcting factors used for determining the set of estimated correcting factors having been obtained under the chosen extraction conditions;

- the set of previous correcting factors has been determined at a first temperature, while the extraction is carried out at a second temperature distinct from the first temperature, the method comprising a step of calculating a set of transposed estimated correcting factors at the second temperature from the set of estimated correcting factors at the first temperature obtained from the previous correcting factors obtained at the first temperature;
- the step of calculating comprises obtaining a set of transposed estimated correcting factors $<p(i,\theta_2)>$ using the following equation:

$$\langle \rho(i,\theta_2) \rangle = 1 + \frac{Q_m}{V_g} \frac{1}{a\,c\,e^{2bF_i}} + \frac{Q_m}{V_m} \frac{1}{c\,e^{bF_i}} + \frac{Q_m}{Q_g} \frac{1}{a\,e^{bF_i}}$$

with the component factor

$$F_i(i,\theta_2) = \frac{\left[\frac{1}{\theta_b(i)} - \frac{1}{\theta_2}\right]}{\left[\frac{1}{\theta_b(i)} - \frac{1}{\theta_c(i)}\right]} log\left(\frac{P_c(i)}{P_{atm}}\right)$$

where parameters a, b and c are constant characteristics of the drilling fluid itself, obtained by a fitting method applied to the set of previous correcting factors $<p(i,\theta_1)>$ at the temperature $\theta_1$, $\theta_b$ is the boiling temperature of the gas compound at atmospheric pressure, $\theta_c$ is the critical temperature of the second compound, $P_c$ is the critical pressure of the gas compound and $P_{atm}$ is the atmospheric pressure, $F_I$ varies with the temperature of the drilling fluid $\theta_1$;
- the method comprises providing an initial set of previous correcting factors obtained from a database, determining a temperature associated with each of the previous correcting factors of the initial set, and calculating a transposed previous correcting factor at the first temperature from a previous correcting factor associated to a temperature distinct from the first temperature, the set of previous correcting factors used for calculating the estimated correcting factors comprising the transposed previous correcting factor;
- the method comprises determining, for each estimated correcting factor, an indicator of the accuracy of the estimated correcting factor depending on the set of previous correcting factors;
- the indicator is one of:

  - a standard deviation a from the set of previous correcting factors;
  - a ratio of the standard deviation over the average $\sigma/<\rho(i)>$;
  - an interval of confidence $IC = 2\sigma/\sqrt{n}$, with $\sigma$ the standard deviation and n the number of values of $p_0(i)$ used In the calculation.

The disclosure also relates to a device for determining the content of several gas compounds contained in a drilling fluid. This device comprises;

- an extractor, able to take out of a sample of the drilling fluid a gas fraction of each gas compound;
- a measuring unit, able to determine a representative information of each gas fraction of each gas compound;
- a computing unit, able to obtain a correcting factor for each gas compound relating the measured information for the gas fraction of each compound to the content of said compound in the drilling fluid;
- a calculation unit estimating the correcting factor used for the or each gas compound on the basis of a set of previous correcting factors previously obtained from calibration of reference drilling fluids, each of the reference drilling fluids having at least a physical property substantially identical to the corresponding physical property of the extracted drilling fluid.

The device according to the disclosure may comprise one or more of the following features, taken individually or according to any technically possible combinations:

- the device comprises a database of correcting factors from previous calibration operations, the calculation unit being adapted to a obtain correcting factors of the set of previous correcting factors from the database;
- the device comprises a determination unit for determining a set of previous correcting factors;
- the device comprises an additional measuring unit, for measuring physical properties of the drilling fluid;
- the device is adapted to carry out the steps of the method mentioned above.

The disclosure will be better understood upon reading the description which follows, given only as an example, and made with reference to the appended drawings, wherein:

- Figure 1 is a schematic vertical sectional view of a drilling installation in which a determination method according to the disclosure is applied;
- Figure 2 is a graphic representation of a set of previous correction factors used to calculate an estimated correction factor, with excluded abnormal values;
- Figure 3 is a graphic representation of estimated correction factors associated with different compounds with their corresponding intervals of confidence, as measured in an oil-based mud at 90°C.
- Figure 4 is a graphic representation of the transposition of a correction coefficient from a first temperature to a second lower temperature.

In all the following, the terms of "upstream" and "downstream" are understood relatively to the normal direction of circulation of a fluid in a conduit.

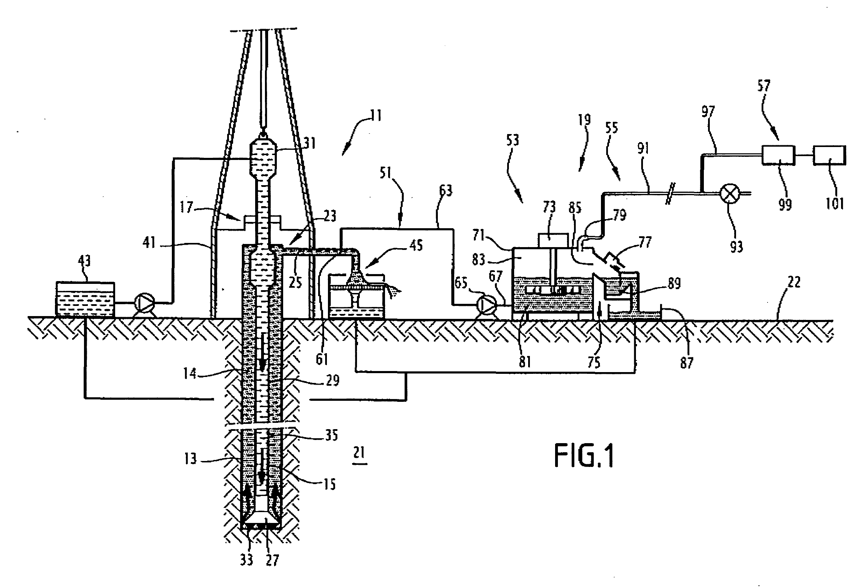

A determination method according to the disclosure Is intended to be applied for analyzing mud gases arising from a drilling installation 11 of a well for producing fluid, notably hydrocarbons, such as an oil well. Such an installation 11 is illustrated by figure 1. This installation 11 comprises a drilling conduit 13 positioned In a cavity 14 pierced by a rotary drilling tool 15, a surface installation 17, and an assembly 19 for analyzing the gases contained in the drilling fluid.

The drilling conduit 13 is positioned in the cavity 14 pierced In the subsoil 21 by the rotary drilling tool 15. It extends in an upper portion of the height of the cavity 14 which it delimits. The cavity 14 further has a lower portion directly delimited by the subsoil. The drilling conduit 13 includes at the surface 22 a well head 23 provided with a conduit 25 for circulation of the drilling fluid. The drilling tool 15 comprises, from bottom to top in figure 1, a drilling head 27, a drill string 29, and a head 31 for injecting drilling fluid. The drilling tool 15 is driven into rotation by the surface installation 17.

The drilling head 27 comprises a tool 33 for piercing the rocks of the subsoil 21. It is mounted on the lower portion of the drill string 29 and is positioned at the bottom of the cavity 14. The drill string 29 comprises a set of hollow drilling tubes. These tubes delimit an inner space 35 which allows the drilling fluid injected through the head 31 from the surface 22 to be brought as far as the drilling head 27. For this purpose, the injection head 31 Is screwed onto the upper portion of the drill string 29. This drilling fluid, commonly designated with the term of "drilling mud", is essentially liquid. The surface installation 17 comprises an apparatus 41 for supporting and driving into rotation the drilling tool 15, an apparatus 43 for injecting the drilling fluid and a vibrating sieve 45. The injection apparatus 43 is hydraulically connected to the injection head 31 for introducing and circulating the drilling fluid in the internal space 35 of the drill string 29.

The drilling fluid is introduced into the inner space 35 of the drill string 29 through the injection apparatus 43. This fluid flows downwards down to the drilling head 27 and passes into the drilling conduit 13 through the drilling head 27. This fluid cools and lubricates the piercing tool 33. The fluid collects the solid debris resulting from the drilling and flows upwards through the annular space defined between the drill string 29 and the walls of the drilling conduit 13, and is then discharged through the circulation conduit 25. The drilling fluid present in the cavity 14 maintains hydrostatic pressure in the cavity, which prevents breakage of the walls delimiting the cavity 14 not covered by the conduit 13 and which further avoids eruptive release of hydrocarbons in the cavity 14.

The circulation conduit 25 is hydraulically connected to the cavity 14, through the well head 23 in order to collect the drilling fluid from the cavity 14. It is for example formed by an open return line or by a closed tubular conduit. In the example illustrated in Fig. 1, the conduit 25 is a closed tubular conduit. The vibrating sieve 45 collects the fluid loaded with drilling residues which flow out of the circulation conduit 25, and separates the liquid from the solid drilling residues. The analysis assembly 19 comprises a device 51 for sampling the drilling fluid In the conduit 25, a device 53 for extracting a gas fraction of the compounds contained in the drilling fluid, a device 55 for transporting extracted gas fractions and an analysis device 57.

The sampling device 51 comprises a sampling head 61 immersed in the circulation conduit 25, a sampling conduit 63 connected upstream to the sampling head 61, a pump 65 connected downstream to the sampling conduit 63, and a supply conduit 67 for bringing the drilling fluid into the extraction device 53, connected to an outlet of the pump 65. The sampling device 51 is further advantageously provided with an assembly for heating the sampled fluid (not shown). This heating assembly is for example positioned between the pump 65 and the extraction device 53 on the supply conduit 67 and enables to heat the drilling fluid at a predetermined temperature at which the extraction of gas compounds from the drilling fluid is easier. The pump 65 is for example a peristaltic pump capable of conveying the drilling fluid sampled by the head 61 towards the extraction device 53 with a determined fluid volume flow rate $Q_m$.

The extraction device 53 comprises an enclosure 71 into which the supply conduit 67 opens out, a rotary stirrer 73 mounted in the enclosure 71, a mud discharge conduit 75, an inlet 77 for injecting a carrier gas and an outlet 79 for sampling the extracted gas fractions in the enclosure 71. The enclosure 71 has an inner volume for example comprised between 0.04 L and 3 L It defines a lower portion 81 of average volume $V_m$, kept constant, in which circulates the drilling fluid stemming from the supply conduit 67 and an upper portion 83 of average volume $V_g$ kept constant and defining a gas head space above the drilling fluid. The mud supply conduit 67 opens out into the lower portion 81. The stirrer 73 is immersed into the drilling fluid present in the lower portion 81. It is capable of vigorously stirring the drilling fluid in order to extract gases therefrom. The discharge conduit 75 extends between an overflow passage 85 made in the upper portion 83 of the enclosure 71 and a retention tank 87 intended to receive the drilling fluid discharged out of the extraction device 53. The discharge conduit 75 is advantageously bent in order to form a siphon 89 opening out facing the retention tank 87 above the level of liquid contained in this tank 87. Alternatively, the drilling fluid from the conduit 75 is discharged into the circulation conduit 25.

In this example, the inlet for Injecting a carrier gas 77 opens out into the discharge conduit 75 upstream from the siphon 89 in the vicinity of the overflow passage 85. Alternatively, the inlet 77 opens out into the upper portion 83 of the enclosure 71. The sampling outlet 79 opens out into an upper wall delimiting the upper portion 83 of the enclosure 71. The drilling fluid introduced into the enclosure 71 via the supply conduit 67 is discharged by overflow into the discharge conduit 75 through the overflow passage 85. A portion of the discharged fluid temporarily lies in the siphon 89 which prevents gases from entering the upper portion 83 of the enclosure 71 through the discharge conduit 75. The Introduction of gas into the enclosure 71 is therefore exclusively carried out through the inlet for injecting a carrier gas 77. In the example illustrated by figure 1, the carrier gas introduced through the introduction inlet 77 is formed by the surrounding air around the installation, at atmospheric pressure. Alternatively, this carrier gas is another gas such as nitrogen or helium.

The transport device 55 comprises a line 91 for transporting the extracted gases towards the analysis device 57 and an apparatus 93 providing suction for conveying the gases extracted out of the enclosure 71 through the transport line 91. The transport line 91 extends between the sampling outlet 79 and the analysis device 57. It advantageously has a length comprised between 10m and 500 m, In order to move the analysis device 57 away from the well head 23 into a non-explosive area. The transport line 91 is advantageously made on the basis of a metal or polymer material, notably polyethylene and/or polytetrafluoroethylene (PTFE).

The analysis device 57 comprises a sampling conduit 97 tapped on the transport line 91 upstream from the apparatus 93 providing suction, an instrumentation 99, and a computing unit 101. The instrumentation 99 is capable of detecting and quantifying the gas fractions extracted out of the drilling fluid in the enclosure 71 which have been transported through the transport line 91. This instrumentation for example comprises infrared detection apparatuses for the amount of carbon dioxide, chromatographs with flame ionization detectors (FID) for detecting hydrocarbons or further with thermal conductivity detectors (TCD) depending on the gases to be analyzed. It may also comprise a chromatography system coupled with a mass spectrometer, this system being designated by the acronym "GC-MS". It may comprise an isotope analysis apparatus as described in Application EP-A-1 887 343 of the Applicant. Online simultaneous detection and quantification of a plurality of compounds contained in the fluid, without any manual sampling by an operator, is therefore possible within time intervals of less than 1 minute.

As this will be seen below, the computing unit 101 is capable of calculating the content of a plurality of compounds to be analyzed present in the drilling fluid, on the basis of the value of the extracted gas fractions in the enclosure 71, as determined by the instrumentation 99, and on the basis of correction factors p(i) specific to each compound to be analyzed and the drilling fluid they are extracted from. The drilling fluid for example is formed by a water-based mud, an oil-based mud or a synthetic-based mud. A water-based mud is a mud having water as a main component (or continuous phase), an oil-based mud is a mud having natural oil as a main component, such as diesel or mineral oil, and a synthetic-based mud is a mud having as main component synthetic oil, such as linear alphaolefins (LAO) or isomerized olefins (IO). Advantageously, the main component of a mud type accounts for over 50% of the volume of the mud. The compounds to be analyzed contained in the drilling fluid are notably aliphatic or aromatic $C_1$-$C_{10}$ hydrocarbons.

As will be seen below, the computing unit comprises a database of correcting factors $\rho_0(i)$ from previous operations, a calculation module able to determine correction factors for the present operation based on the mud type and on previous correction factors $\rho_0(i)$ stored on the database and an analysis module for determining the content of each compound in the mud based on a measured value of the compound concentration and on the correction factor p(i). A mud type may be a water-based mud, an oil-based mud or a synthetic-based mud or for instance a first type of mud may be water-based mud and a second type of mud may be a group comprising oil-based mud and synthetic-based mud.

The application of a first determination method according to the disclosure will now be described. This method comprises an evaluation of the correction factors $\rho(i)$ of a group of compounds i to be analyzed at a temperature $\theta_1$. It may also comprise the transposition of said correction factors to the operating temperature $\theta_2$ of the drilling fluid in the enclosure 71, and then an online analysis, applied during the drilling, of the content of said compounds in the drilling fluid circulating in the circulation conduit 25.

The determination of the correcting factors p(i) of a group of compounds i to be analyzed starts with the determination of the type of the drilling fluid currently used in the current operation from a given number of known types, including in particular a water-based drilling mud, an oil-based drilling mud and a synthetic-based drilling mud.

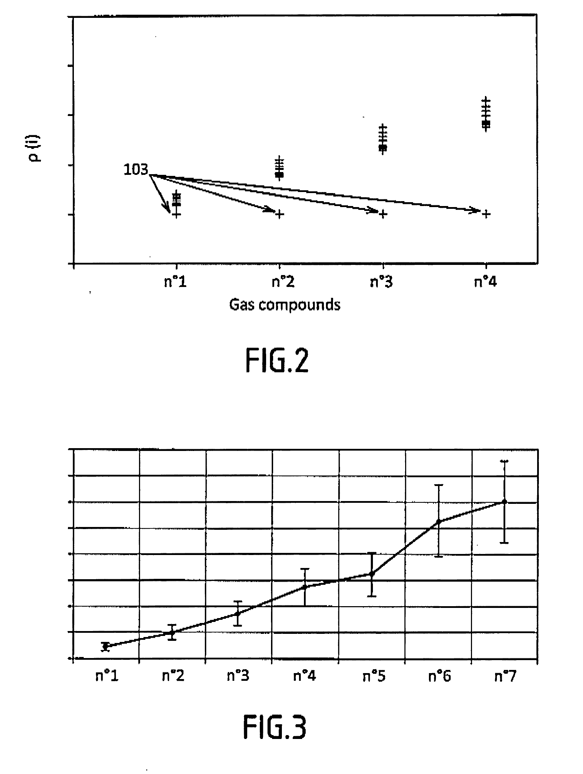

Previous correcting factors $\rho_0(i)$ associated to the same compound and the same type of drilling fluid are then extracted from a database containing previous results for each possible drilling fluid type, obtained In previous calibration operations, as illustrated in figure 2. The previous calibrations may have been performed at a wellsite and/or in a lab.

Abnormal values 103 of previous correcting factors $\rho_0(i)$ are excluded from this data, including for example coefficients equal to 1, which would represent a complete extraction of the gas compound from the mud. Such abnormal values are attributed to experimentation errors or input errors in the database, and as such, the values of all the coefficients associated to each of the gas compounds obtained in this same previous operation are expected to have been impacted by said errors.

The search of abnormal values may be conducted on the basis of values of the previous correcting factor $\rho_0(i)$ associated to only one first gas compound i, in this example $C_1$, and the associated correcting factors from the same abnormal operation, corresponding to other compounds $C_1$, are excluded if the value associated to the first compound is abnormal.

An estimated correction coefficient $<\rho(i)>$ is then calculated for each compound by calculating the average value of the corresponding previous correcting factors $\rho_0(i)$ from the database after the exclusion of abnormal values.

This calculation is completed by calculating other statistical coefficients, such as standard deviation $\sigma$, the ratio of said standard deviation over the average $\sigma/<\rho(i)>$, and the interval of confidence $IC = 2\sigma/\sqrt{n}$, with n the number of values of $\rho_0(i)$ used in the calculation. This additional statistical information is used to evaluate the accuracy of the approximation. This process is illustrated on figure 3, which shows the averaged values $<\rho(i)>$ associated with seven compounds as calculated with the described method, with corresponding intervals of confidence.

All the values from the database have been obtained from calibration operations at a first fixed temperature $\theta_1$, depending on the type of drilling fluid. For example, water-based muds (WBM) are measured at 70°C, while oil-based muds (OBM) and synthetic-based muds (SBM) are measured at 90°C.

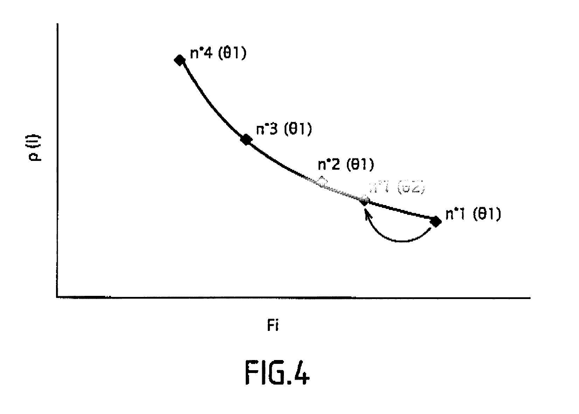

In order to accommodate the operating conditions of an-on field extraction, the value of the correcting factors $<\rho(i,\theta_1)>$ used have to be transposed to the temperature $\theta_2$ of the drilling fluid in the enclosure 71, following a method as follow, represented in figure 4.

A thermodynamic factor $F_I$ specific to each compound, depending on the temperature of the drilling fluid $\theta_1$ during the extraction is calculated for each of the averaged correcting coefficients $<\rho(i,\theta_1)>$ associated to each compound calculated from values out of the database.

The coefficient $F_I$ is advantageously taken as proposed by Hoffman (Hoffman et al. «Equilibrium Constants for a Gas Condensate System» Trans. AIME (1953) 198, 1-10) or in an improved way by Standing (Standing, <<A set of Equations for Computing Equilibrium Ratios of a Crude Oil/Natural Gas System at Pressures below 1,000 psia>> SPE 7903 1979). It is calculated with the following equation:

$$F_i(i,\theta_1) = \frac{\left[\frac{1}{\theta_b(i)} - \frac{1}{\theta_1}\right]}{\left[\frac{1}{\theta_b(i)} - \frac{1}{\theta_c(i)}\right]} \log\left(\frac{P_c(i)}{P_{atm}}\right) \tag{1}$$

Where $\theta_1$ is the temperature of the drilling fluid, $\theta_b(i)$ is the boiling temperature at atmospheric pressure of the compound i, $\theta_c(i)$ is the critical temperature of compound i, Pc(i) is the critical pressure of compound i and $P_{atm}$ is the

atmospheric pressure.

The couples of values ($<\rho(i,\theta_1)>$, $F_l$) associated to each of the compounds i are then used to lay out a system of equations as described in US 2011/303463. Advantageously, the equations binding each pair ($<\rho(i,\theta_1)>$, $F_l$) are of the following form:

$$\langle \rho(i, \theta_1) \rangle = 1 + \frac{Q_m}{V_g} \frac{1}{a\, c\, e^{2bF_l}} + \frac{Q_m}{V_m} \frac{1}{c\, e^{bF_l}} + \frac{Q_m}{Q_g} \frac{1}{a\, e^{bF_l}} \qquad (2)$$

The parameters a, b and c only depend on the type of drilling fluid considered and are the same for each gas compound considered, as well as for each temperature of the drilling fluid. The parameters a, b and c can thus be determined by solving the previous system of equations by an optimization method, for example the least squares method.

Once a, b and c are determined for the type of drilling fluid currently in operation, the value of the correction factors $<\rho(i,\theta_2)>$ associated to each gas compound can be calculated at the effective operating temperature $\theta_2$ of the drilling fluid in the enclosure 71 by calculating $F_l(i,\theta_2)$.

An example of this temperature transposition is shown on figure 4, where the average values of the correcting coefficient $<\rho(i,\theta_1)>$ associated with four different compounds obtained at a first temperature $\theta_1$ are used to calculate the parameters a, b and c, which in turn are used to draw the represented curve. This curve is then used to calculate the transposed value of $<\rho(n°1,\theta_2)>$ at a second temperature $\theta_2$, based on the average value $<\rho(n°1,\theta_1)>$ determined from the database at a first temperature $\theta_1$.

If every previous calibration operation has not been performed at the same first temperature, similar calculations may be performed on at least one of the previous correcting factors to transpose the previous correcting factor at the first temperature so that all the previous correcting factors of the set used for the estimation are determined at the same temperature, which is the first temperature $\theta_1$.

The analysis is then applied during the drilling. In order to carry out the drilling, the drilling tool 15 is driven into rotation by the surface installation 41. The drilling fluid is introduced into the inner space 35 of the drilling lining 29 through the injection apparatus 43. This fluid flows down to the drilling head 27 and passes in the drilling conduit 13 through the drilling head 27. This fluid cools and lubricates the piercing tool 33. The fluid collects solid debris resulting from the drilling and moves up through the annular space defined between the drill string 29 and the walls of the drilling conduit 13, and is then discharged through the circulation conduit 25.

In this step, the sampling head 61 is positioned in the circulation conduit 25, downstream from the vibrating sieve 45. The pump 65 is then actuated in order to pick up drilling fluid in the conduit 25 with the given volume flow rate $Q_m$ and to introduce it into the enclosure 71 through the admission conduit 67. The drilling fluid then contains the components to be analyzed. The stirrer 73 is actuated for stirring the drilling fluid present in the lower portion 81 and for extracting a gas fraction y(i) of each compound i present in the drilling fluid. This gas fraction y(i) is conveyed at a total flow rate $Q_g$ as far as the instrumentation 99 through the transport line 91 in order to determine its value. The calculation unit 101 uses all the calculated correction coefficients $\rho_0(i)$ associated with the required gas compounds associated to the type of drilling fluid in use on the drilling operation, at the temperature at which the drilling fluid enter the enclosure 71.

The computing unit 101 infers therefrom the value of the content *x(i)* of each compound I in the drilling fluid by equation (3), using the averaged value $<\rho(1,\theta_2)>$ of the correcting coefficient, transposed at the effective temperature $\theta_2$ of the drilling fluid in the enclosure 71:

$$x(i) = \frac{Q_g}{Q_m} \langle \rho(i, \theta_2) \rangle\, y(i) \qquad (3)$$

With the method according to the disclosure, it is possible to obtain a quantification of the gases dissolved in the drilling fluid with a relatively good accuracy without performing a time-consuming calibration and thus making the whole process shorter and simpler to execute. It allows to constructively the results from previous calibration operations and improves the efficiency in the field environment.

The method according to the disclosure may not comprise a transposition step. The correcting coefficients at the current operation temperature $\theta_2$ are obtained from values $\rho_0(i)$ arising from operations at the same temperature $\theta_2$ in the database.

The method according to the disclosure may also determine the set of previous correcting factors not only on the basis of the type of the drilling fluid but on any physical property of the drilling fluid (such as density or viscosity for example). In this case, the reference drilling fluids involved In the calibration of the previous correction factor may be chosen so that the difference between the value of said physical property for the extracted drilling fluid and reference drilling fluid is contained in a predetermined range, for instance close to 0, or so that a ratio of the values of said physical property for the extracted drilling fluid and reference drilling fluid Is contained in a predetermined range, for instance close to 1, for example between 0.9 and 1.1.

The value of said physical property may be well-known, or it may be measured during drilling, for instance at the exit of the well or before injecting the drilling mud in the rig, by any conventional means. The measurement may be performed In real-time so as to change the set of previous correcting factors used for the estimation in real-time.

The specific embodiments described above have been shown by way of example, and it should be understood that these embodiments may be susceptible to various modifications and alternative forms. It should be further understood that the claims are not intended to be limited to the particular forms disclosed, but rather to cover modifications, equivalents, and alternatives falling within the spirit and scope of this disclosure.

**Claims**

1. A method for determining the content of at least one gas compound contained in a drilling fluid having a set of physical properties, the method comprising:

   - extracting out of a sample of the drilling fluid a gas fraction of the or each gas compound;
   - measuring a representative information ($y(i)$) of the or each gas fraction of each gas compound;
   - obtaining, for the or each gas compound a correcting factor ($<\rho(i)>$) relating the measured information ($y(i)$) for the gas fraction of each compound to the content of said compound in the drilling fluid;

   wherein the estimated correcting factor ($<\rho(i)>$) used for the or each gas compound is obtained on the basis of a set of previous correcting factors ($\rho_0(i)$), previously obtained from calibration of reference drilling fluids, each of the reference drilling fluids having at least one physical property substantially identical to the corresponding physical property of the extracted drilling fluid.

2. The method according to claim 1, comprising determining the set of previous correcting factors ($\rho_0(i)$).

3. The method according to claim 1 or 2, wherein the set of previous correcting factors ($\rho_0(i)$) is chosen so as to have been calibrated for the same gas compound.

4. The method according to any one of the preceding claims, wherein the or one of the physical property relates to the composition of the drilling fluid, wherein the reference drilling fluids are of the same type as the extracted drilling fluid, wherein the type is optionally chosen among a water-based mud, an oil-based mud or a synthetic-based mud.

5. The method according to any one of the preceding claims, wherein the or one of the physical property is density or a rheological property such as viscosity, wherein the difference between the value of the physical property for each of the reference drilling fluids and the value of the corresponding physical property for the extracted drilling fluid is situated in a predetermined range.

6. The method according to any of the preceding claims, comprising measuring at least a physical property of the drilling fluid.

7. The method according to any one of the preceding claims, wherein the estimated correcting factor ($<\rho(i)>$) for a gas compound corresponds to the average value calculated over the set of previous correcting factors related to the same compound.

8. The method according to any one of the preceding claims, comprising providing an initial set of previous correcting factors obtained from a database, determining a number of abnormal values from this initial set and determining the set of previous correcting factors ($\rho_0(i)$) used for calculating the estimated correcting factors by excluding the abnormal values.

9. The method according to any one of the preceding claims, wherein the extraction is implemented on an enclosure comprising a stirrer for stirring the drilling fluid, with chosen extraction conditions comprising at least one of the following:

   - a flow rate of injection of the drilling fluid in the enclosure $Q_m$,
   - an average volume of drilling fluid in the enclosure $V_m$,

- a volume of overhead gas in the enclosure $V_g$,
- a flow rate of gas extracted from the drilling fluid $Q_g$.

the set of previous correcting factors ($\rho_0(i)$) used for determining the set of estimated correcting factors ($<\rho(i)>$) having been obtained under the chosen extraction conditions.

10. The method according to claim 9, wherein the set of previous correcting factors have been determined at a first temperature ($\theta_1$), while the extraction is carried out at a second temperature ($\theta_2$) distinct from the first temperature ($\theta_1$), the method comprising a step of calculating a set of transposed estimated correcting factors at the second temperature ($\theta_2$) from the set of estimated correcting factors at the first temperature ($\theta_1$) obtained from the previous correcting factors obtained at the first temperature ($\theta_1$).

11. The method according to claim 10, wherein the step of calculating comprises obtaining at least one transposed estimated correcting factors $<\rho(i,\theta_2)>$ using the following equation:

$$\langle\rho(i,\theta_2)\rangle = 1 + \frac{Q_m}{V_g}\frac{1}{a\,c\,e^{2bF_i}} + \frac{Q_m}{V_m}\frac{1}{c\,e^{bF_i}} + \frac{Q_m}{Q_g}\frac{1}{a\,e^{bF_i}}$$

with the component factor

$$F_i(i,\theta_2) = \frac{\left[\frac{1}{\theta_b(i)} - \frac{1}{\theta_2}\right]}{\left[\frac{1}{\theta_b(i)} - \frac{1}{\theta_c(i)}\right]}log\left(\frac{P_c(i)}{P_{atm}}\right)$$

where parameters a, b and c are constant characteristics of the drilling fluid itself, obtained by a fitting method applied to the set of previous correcting factors $<\rho(i,\theta_0)>$ at the temperature $\theta_1$, $\theta_b$ is the boiling temperature of the gas compound at atmospheric pressure, $\theta_c$ is the critical temperature of the second compound, $P_c$ is the critical pressure of the gas compound and $P_{atm}$ is the atmospheric pressure, $F_l$ varies with the temperature of the drilling fluid $\theta_1$.

12. The method according to any of the preceding claims, comprising providing an initial set of previous correcting factors obtained from a database, determining a temperature associated with each of the previous correcting factors of the initial set, and calculating a transposed previous correcting factor at the first temperature from a previous correcting factor associated to a temperature distinct from the first temperature, the set of previous correcting factors ($\rho_0(i)$) used for calculating the estimated correcting factors comprising the transposed previous correcting factor.

13. The method according to any of the.preceding claims, comprising determining, for each estimated correcting factor ($<p(i)>$), at least one indicator of the accuracy of the estimated correcting factor depending on the set of previous correcting factors.

14. The method of the preceding claim, wherein the at least one indicator comprises at least one of:

- a standard deviation $\sigma$ from the set of previous correcting factors;
- a ratio of the standard deviation over the average $\sigma/<\rho(i)>$;
- an interval of confidence $IC=2\sigma/\sqrt{n}$, with $\sigma$ the standard deviation and n the number of values of $\rho_0(i)$ used in the calculation.

15. A device for determining the content of at least one gas compound contained in a drilling fluid having a set of physical properties, the device comprising:

- an extractor, able to take out of a sample of the drilling fluid a gas fraction of the or each gas compound;
- a measuring unit, able to determine a representative information (y(i)) of the or each gas fraction of each gas compound;
- a computing unit, able to obtain a correcting factor ($<\rho(i)>$) for the or each given gas compound relating the measured information (y(i)) for the gas fraction of each compound to the content of said compound in the drilling fluid;

wherein the computing unit comprises a calculation unit estimating the correcting factor ($<\rho(i)>$) used for the or each gas compound on the basis of a set of previous correcting factors ($\rho_0(i)$) previously obtained from calibration of reference drilling fluids, each of the reference drilling fluids having at least a physical property substantially identical to the corresponding physical property of the extracted drilling fluid.

FIG.1

FIG.2

FIG.3

FIG.4

Europäisches
Patentamt

European
Patent Office

Office européen
des brevets

## EUROPEAN SEARCH REPORT

Application Number

EP 15 29 0274

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 703 597 A1 (GEOSERVICES EQUIPEMENTS [FR]) 5 March 2014 (2014-03-05) | 1,3,5,6, 9-15 | INV. G01N33/28 |
| Y | * abstract; claims 1,8 * <br> * pages 9,10 * | 2,4,7,8 | |
| X | US 2010/089120 A1 (HANSON SCOTT A [US]) 15 April 2010 (2010-04-15) | 1,3,5,6, 13-15 | |
| Y | * abstract; figures 1,3,5 * <br> * paragraphs [0006], [0007], [0017], [0018], [0024], [0032] * | 4 | |
| Y | US 4 887 464 A (TANNENBAUM ELI [IL] ET AL) 19 December 1989 (1989-12-19) | 2,7,8 | |
| A | * column 8, line 5 - line 49 * | 12 | |

TECHNICAL FIELDS
SEARCHED      (IPC)

G01N

The present search report has been drawn up for all claims

| Place of search | Date of completion of the search | Examiner |
|---|---|---|
| The Hague | 17 February 2016 | Steinmetz, Johannes |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 29 0274

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2016

| Patent document cited in search report | | Publication date | Patent family member(s) | | Publication date |
|---|---|---|---|---|---|
| EP 2703597 | A1 | 05-03-2014 | EP | 2703597 A1 | 05-03-2014 |
| | | | US | 2014067307 A1 | 06-03-2014 |
| | | | WO | 2014033265 A1 | 06-03-2014 |
| US 2010089120 | A1 | 15-04-2010 | AU | 2009302654 A1 | 15-04-2010 |
| | | | BR | PI0919573 A2 | 08-12-2015 |
| | | | CA | 2739593 A1 | 15-04-2010 |
| | | | CN | 102216563 A | 12-10-2011 |
| | | | EP | 2340354 A2 | 06-07-2011 |
| | | | RU | 2011118473 A | 20-11-2012 |
| | | | US | 2010089120 A1 | 15-04-2010 |
| | | | WO | 2010042383 A2 | 15-04-2010 |
| US 4887464 | A | 19-12-1989 | DE | 68918408 D1 | 27-10-1994 |
| | | | EP | 0370548 A1 | 30-05-1990 |
| | | | NO | 894367 A | 23-05-1990 |
| | | | US | 4887464 A | 19-12-1989 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2799790
- EP 1710575 A
- EP 1887343 A
- US 2011303463 A

**Non-patent literature cited in the description**

- **HOFFMAN et al.** Equilibrium Constants for a Gas Condensate System. *Trans. AIME,* 1953, vol. 198, 1-10